# EUROPEAN PATENT SPECIFICATION

(11) **EP 3 865 179 B1**
(45) Date of publication and mention of the grant of the patent: **23.07.2025**
(21) Application number: 19875071.3
(22) Date of filing: 25.10.2019
(51) Int. Cl.: A61K 38/17, A61P 19/00, A61P 19/02

(54) **HMGB1 DERIVED PEPTIDE FOR TREATING OR PREVENTING A CARTILAGE DISORDER**
HMGB1-ABGELEITETES PEPTID ZUR BEHANDLUNG ODER VORBEUGUNG EINER KNORPELERKRANKUNG
PEPTIDE DÉRIVÉ DE HMGB1 POUR TRAITER OU PRÉVENIR UN TROUBLE DU CARTILAGE

(30) Priority: 25.10.2018 JP 2018200866
(43) Date of publication of application: 18.08.2021
(73) Proprietor: The University of Osaka, Suita-shi, Osaka 565-0871 (JP); Stemrim Inc., Ibaraki-shi, Osaka 567-0085 (JP)
(72) Inventor: TAMAI, Katsuto, Suita-shi, Osaka 565-0871 (JP); SHIMBO, Takashi, Suita-shi, Osaka 565-0871 (JP); SASAKI, Eiji, Suita-shi, Osaka 565-0871 (JP); TSUSHIMA, Takahiro, Suita-shi, Osaka 565-0871 (JP); YAMAZAKI, Takehiko, Ibaraki-shi, Osaka 567-0085 (JP)
(74) Representative: Moré, Solveig Helga
(86) International application number: PCT/JP2019/042015
(87) International publication number: WO 2020/085506

(56) References cited:
- EP-A1- 3 719 117
- EP-A1- 3 750 553
- WO-A1-2012/147470
- WO-A1-2014/065348
- WO-A1-2018/199107
- JP-A- 2010 503 630
- US-A1- 2004 156 851
- US-A1- 2008 038 309
- NOBORU TANIGUCHI , KENJI YOSHIDA , TATSUO ITO , MASANAO TSUDA , YASUNORI MISHIMA , TAKAYUKI FURUMATSU , LORENZA RONFANI , KAZUHIRO: "Stage-specific secretion of HMGB1 in cartilage regulates endochondral ossification", MOLECULAR AND CELLULAR BIOLOGY, vol. 27, no. 16, 1 August 2007 (2007-08-01), pages 5650 - 63, XP055709081, ISSN: 0270-7306, DOI: 10.1128/MCB.00130-07
- TAMAI KATSUTO: "Chapter 2 Various disease models and drug discovery research, 7. Skin and inflammatory diseases, 2) Prospects for the development of in vivo regenerative induction drug using in vivo bone marrow mesenchymal stem/ progenitor cell mobilization factor", GENE & MEDICINE, vol. 22, 22 July 2012 (2012-07-22), JP, pages 207 - 212, XP008179446, ISSN: 1349-2527
- GEOFFREY LEE, ANA ISABEL ESPIRITO SANTO, STEFAN ZWINGENBERGER, LAWRENCE CAI, THOMAS VOGL, MARC FELDMANN, NICOLE J. HORWOOD, JAMES : "Fully reduced HMGB1 accelerates the regeneration of multiple tissues by transitioning stem cells to GAlert", PROCEEDINGS OF THE NATIONAL ACADEMY OF SCIENCES, vol. 115, no. 19, 8 May 2018 (2018-05-08), pages E4463 - E4472, XP055621911, ISSN: 0027-8424, DOI: 10.1073/pnas.1802893115
- KATSURA SUGAWARA: "Artificial organ-Recent advance, Recent advance in cartilage loss treatment by cultured cartilage", JINKO ZOKI, vol. 42, no. 3, 15 December 2013 (2013-12-15), pages 198 - 200, XP055806414, ISSN: 0300-0818, DOI: 10.11392/jsao.42.198
- JULIEN FREITAG , KIRAN SHAH, JAMES WICKHAM, RICHARD BOYD, ABI TENEN: "The effect of autologous adipose derived mesenchymal stem cell therapy in the treatment of a large osteochondral defect of the knee following unsuccessful surgical intervention of osteochondritis dissecans - a case study", BMC MUSCULOSKELET DISORD., vol. 18, no. 1, 1 December 2017 (2017-12-01), pages 1 - 11, XP055709084, DOI: 10.1186/s12891-017-1658-2

## Description

### Technical Field

The present application relates to a pharmaceutical composition for preventing and/or treating a cartilage disorder, containing a fragment peptide of a high mobility group box 1 (HMGB1) protein. This application claims priority to Japanese Patent Application No. 2018-200866, filed on October 25, 2018.

### Background Art

Cartilage tissue such as articular cartilage is less repairable, and almost impossible to naturally regenerate once it is damaged. For example, therapies for traumatic articular cartilage defect are mainly symptomatic therapies such as oral administration of analgesics and intraarticular injection of hyaluronic acid, and no curative therapy for traumatic articular cartilage defect has yet been established. In addition, there are surgical treatment methods such as autologous osteochondral column transplantation for traumatic articular cartilage defect, but they have problems such as the inability to cope with large defects because of the need to collect normal tissues of the patient. Thus, it is desired to develop a more effective and less invasive therapeutic agent for cartilage disorders.

### Citation List

### Patent Literature

Patent Literature 1: WO2012/147470
Patent Literature 2: WO2014/065347
Patent Literature 3: WO2014/065348
Patent Literature 4: WO2018/139562
Patent Literature 5: WO2018/186480

### Summary of Invention

### Technical Problem

An object of the present application is to provide a novel medicament effective for treating a cartilage disorder.

### Solution to Problem

The present inventors have searched for a substance effective for treating a cartilage disorder, and consequently have found that an HMGB1 fragment peptide having a particular amino acid sequence have an effect of regenerating normal cartilage tissue including hyaline cartilage in an animal model with articular cartilage defect. Based on this finding, the present application provides a pharmaceutical composition for preventing and/or treating a cartilage disorder, containing the specific HMGB1 fragment peptide.

That is, the present application provides the following:
[1] A pharmaceutical composition for use in preventing and/or treating a cartilage disorder, comprising any one substance of (a) to (c) (hereinafter referred to as substance A):
   (a) an HMGB1 fragment peptide consisting of an amino acid sequence set forth in SEQ ID NO: 1;
   (b) a peptide consisting of an amino acid sequence set forth in SEQ ID NO: 1 with one or more amino acids substituted, deleted, inserted, or added; and
   (c) a peptide consisting of an amino acid sequence having a sequence identity of about 80% or more to the amino acid sequence set forth in SEQ ID NO: 1.
[2] The pharmaceutical composition according to [1], wherein the cartilage disorder is traumatic cartilage defect, osteoarthritis, or osteochondritis dissecans.
[3] A pharmaceutical composition for use in regenerating hyaline cartilage in a patient with a cartilage disorder, comprising a substance A.
[4] The pharmaceutical composition according to [3], wherein the cartilage disorder is traumatic cartilage defect, osteoarthritis, or osteochondritis dissecans.
[B1] A substance A for use in preventing and/or treating a cartilage disorder.
[B2] The substance A according to [B1], wherein the cartilage disorder is traumatic cartilage defect, osteoarthritis, or osteochondritis dissecans.
[B3] A substance A for use in regenerating hyaline cartilage in a patient with a cartilage disorder.
[B4] The substance A according to [B3], wherein the cartilage disorder is traumatic cartilage defect, osteoarthritis, or osteochondritis dissecans.

[Figure 1] Figure 1 is photographs showing the results of microscopic observations at and around the cartilage defect sites of knee joints at 2, 4, 8 and 12 weeks post-operative ("Vehicle" indicates the control group and "1-44" indicates the HMGB1 peptide (1-44) administration group, respectively).
[Figure 2] Figure 2 is a graph showing the scores of gross appearance by the Wayne scoring system of the cartilage defect sites of knee joints at 2, 4, 8 and 12 weeks post-operative ("Vehicle" shows the control group and "1-44" shows the HMGB1 peptide (1-44) administration group, respectively). The vertical axis shows the score and the horizontal axis shows the number of weeks after creation of the cartilage defect. * p<0.05 (Two-tailed unpaired t-test), † p<0.05 (Two-way ANOVA with post-hoc Tukey's test)
[Figure 3] Figure 3 is photographs showing the results of safranin O staining of the cartilage tissue sections of knee joints at 2, 4, 8 and 12 weeks post-operative ("Vehicle" indicates the control group and "1-44" indicates the HMGB1 peptide (1-44) administration group, respectively).
[Figure 4] Figure 4 is a graph showing the Wakitani scores of the cartilage tissues of knee joints at 2, 4, 8 and 12 weeks post-operative ("Vehicle" indicates the control group and "1-44" indicates the HMGB1 peptide (1-44) administration group, respectively). The vertical axis shows the score and the horizontal axis shows the number of weeks after creation of the cartilage defect. * p<0.05 (Two-tailed unpaired t-test), t p<0.05 (Two-way ANOVA with post-hoc Tukey's test)
[Figure 5] Figure 5 is photographs showing the results by individual of safranin O staining of the cartilage tissue sections of knee joints at 12 weeks post-operative ("Vehicle" indicates the control group and "1-44" indicates the HMGB1 peptide (1-44) administration group, respectively).
[Figure 6] Figure 6 is a graph showing the Wakitani scores by item of the cartilage tissues of knee joints at 12 weeks post-operative ("Vehicle" indicates the control group and "1-44" indicates the HMGB1 peptide (1-44) administration group, respectively). The vertical axis shows the score and the horizontal axis shows the items that constitute the Wakitani score. * p<0.05 (Two-tailed unpaired t-test)
[Figure 7] Figure 7 is photographs showing staining results of the cartilage tissue sections of knee joints at 12 weeks post-operative in the cartilage defect model mice. Left shows the result of safranin O staining, and right shows the result of immunostaining of type II collagen.
[Figure 8] Figure 8 is photographs showing staining results of the cartilage tissue sections of knee joints of normal mice. Left shows the result of safranin O staining, and right shows the result of immunostaining of type II collagen.

### Description of Embodiments

The present application provides a pharmaceutical composition for preventing and/or treating a cartilage disorder, containing an HMGB1 fragment peptide comprising an amino acid sequence set forth in SEQ ID NO: 1.

The cartilage disorder refers to a disorder involving abnormalities of cartilage tissue. Examples of the abnormalities of cartilage tissue include damage, wear, defects, and degeneration of cartilage.

Examples of the cartilage disorder in the present application include, but are not limited to, traumatic cartilage defect, osteoarthritis, osteochondritis dissecans, meniscal damage, traumatic arthritis, inflammatory arthritis (e.g., rheumatoid arthritis), and infectious arthritis (e.g., suppurative arthritis). Osteoarthritis includes primary osteoarthritis for which the cause is unclear and secondary osteoarthritis for which the cause is clear. Examples of the secondary osteoarthritis include, but are not limited to, osteoarthritis caused by ligament damage, cartilage damage, meniscal damage, or the like.

In one preferred aspect, the cartilage disorder treated with the HMGB1 fragment peptide of the present application is traumatic cartilage defect, osteoarthritis, or osteochondritis dissecans. In another preferred aspect, the cartilage disorder treated with the HMGB1 fragment peptide of the present application is traumatic cartilage defect or osteoarthritis. In a further aspect, the cartilage disorder treated with the HMGB1 fragment peptide of the present application is traumatic cartilage defect. In another aspect, the cartilage disorder treated with the HMGB1 fragment peptide of the present application is osteoarthritis.

Examples of the type (site) of cartilage treated with the HMGB1 fragment peptide of the present application include, but are not limited to, cartilage of joint. Examples of the joint include, but are not limited to, extremity joint (shoulder joint, elbow joint, hand joint, hip joint, knee joint, ankle joint), jaw joint, and intervertebral joint.

In one aspect, the cartilage disorder treated with the HMGB1 fragment peptide of the present application is a cartilage disorder of extremity joint. In another aspect, the cartilage disorder treated with the HMGB1 fragment peptide of the present application is a cartilage disorder of elbow joint or knee joint. In a further aspect, the cartilage disorder treated with the HMGB1 fragment peptide of the present application is a cartilage disorder of knee joint.

Further examples of the cartilage disorder treated with the HMGB1 fragment peptide of the present application can include traumatic cartilage defect of joint, traumatic cartilage defect of elbow joint, traumatic cartilage defect of knee joint, elbow osteoarthritis, knee osteoarthritis, osteochondritis dissecans of joint, osteochondritis dissecans of elbow joint, and osteochondritis dissecans of knee joint. In one aspect, the cartilage disorder treated with the HMGB1 fragment peptide of the present application is traumatic cartilage defect of knee joint. In another aspect, the cartilage disorder treated with the HMGB1 fragment peptide of the present application is knee osteoarthritis.

In the present application, the term "pharmaceutical composition" is used interchangeably with the term "medicament", "agent" or "medical composition".

The present application also provides a pharmaceutical composition for use in regenerating hyaline cartilage in a patient with a cartilage disorder, containing an HMGB1 fragment peptide consisting of the amino acid sequence set forth in SEQ ID NO: 1. In one aspect, the pharmaceutical composition of the present application is for use in regenerating hyaline cartilage in a patient with traumatic cartilage defect, osteoarthritis, or osteochondritis dissecans.

In the present application, the HMGB1 fragment peptide consisting of an amino acid sequence set forth in SEQ ID NO: 1 means a peptide consisting of a portion of an HMGB1 protein, wherein the peptide consists of the amino acid sequence set forth in SEQ ID NO: 1. Such a peptide can be obtained by incorporating a DNA encoding the peptide into an appropriate expression system to prepare a recombinant, or can be synthesized artificially.

Examples of the HMGB1 protein in the present application include, but are not limited to, a protein comprising an amino acid sequence set forth in SEQ ID NO: 2 and a protein encoded by a DNA containing a nucleotide sequence set forth in SEQ ID NO: 3.

Examples of the HMGB1 fragment peptide consisting of the amino acid sequence set forth in SEQ ID NO: 1 in the present application include, but are not limited to, an HMGB1 fragment peptide consisting of the amino acid sequence set forth in SEQ ID NO: 1.

In the pharmaceutical composition of the present application, alternatively or additionally to the HMGB1 fragment peptide consisting of the amino acid sequence set forth in SEQ ID NO: 1, a peptide consisting of an amino acid sequence set forth in SEQ ID NO: 1 with one or more amino acid residues modified (substituted, deleted, inserted or added), wherein the peptide is functionally equivalent to the HMGB1 fragment peptide consisting of the amino acid sequence set forth in SEQ ID NO: 1, can be used. Examples of such peptide include, but are not limited to, the following:
i) a peptide consisting of an amino acid sequence set forth in SEQ ID NO: 1 with one to ten (e.g., 1 to 10, 1 to 9, 1 to 8, 1 to 7, 1 to 6, 1 to 5, 1 to 4, 1 to 3, or 1 to 2) amino acids substituted, deleted, inserted, or added;
ii) a peptide consisting of an amino acid sequence having a sequence identity of about 80% or more, e.g., about 85% or more, about 90% or more, about 91% or more, about 92% or more, about 93% or more, about 94% or more, about 95% or more, about 96% or more, about 97% or more, or about 98% or more to the amino acid sequence set forth in SEQ ID NO: 1.

An effective amount of the peptide or a pharmaceutical composition containing the same (hereinafter referred to as the peptide or the like) of the present application is administered to a subject for the treatment or prevention of a disorder or symptom described herein.

An effective amount in the present application refers to an amount sufficient to treat or prevent the disorder or symptom described herein. Examples of the treatment in the present application include, but are not limited to, alleviation, delay, arrest, amelioration, remission, cure, and complete remission. Examples of the prevention in the present application include, but are not limited to, alleviation, delay, and arrest.

The subject in the present application is not particularly limited, and examples thereof include a mammal, a bird, and a fish. Examples of the mammal include, but are not limited to, human and a non-human animal, e.g., human, mouse, rat, monkey, pig, dog, rabbit, hamster, guinea pig, horse, sheep, or whale. In the present application, the term "subject" is used interchangeably with the term "patient", "individual", or "animal."

The administration site of the peptide or the like of the present application is not limited, and the peptide or the like of the present application can exert its effect at wherever site it is administered, such as a site where a symptom of a cartilage disorder appears or a vicinity thereof, a site different from these (a site other than these), a site remote from a site where a symptom of a cartilage disorder appears, a site distal from a site where a symptom of a cartilage disorder appears, or a site distal and ectopic from a site where a symptom of a cartilage disorder appears.

The peptide or the like of the present application can also exert its effect at whichever tissue it is administered, such as a tissue different from a tissue in which a symptom of a cartilage disorder appears (e.g., a joint), a tissue remote from a tissue in which a symptom of a cartilage disorder appears, a tissue distal from a tissue in which a symptom of a cartilage disorder appears, or a tissue distal and ectopic from a tissue in which a symptom of a cartilage disorder appears.

Examples of the administration method of the peptide or the like of the present application include oral administration and parenteral administration, and examples of the parenteral administration method include, but are not limited to, intravascular administration (intraarterial administration, intravenous administration, and the like), intramuscular administration, subcutaneous administration, intradermal administration, intraperitoneal administration, transnasal administration, transpulmonary administration, and transdermal administration. The peptide or the like of the present application can also be administered systemically or topically (e.g., subcutaneously, intradermally, on the surface of the skin, to the eyeball or eyelid conjunctiva, nasal mucosa, intraorally, and to gastrointestinal mucosa, vaginal/intrauterine mucosa, or an injury site) by injection, e.g., intravenous injection, intramuscular injection, intraperitoneal injection, or subcutaneous injection.

Alternatively to the peptide or the like of the present application, a cell that secretes the peptide of the present application, a DNA encoding the peptide, a vector having the DNA inserted therein, a cell containing the vector, and a pharmaceutical composition containing the same can be used.

The administration method can be appropriately selected depending on the age and symptoms of the patient. When the peptide of the present application is administered, the administration amount can be selected, for example, in the range of about 0.0000001 mg to about 1000 mg per kg of body weight per administration. Alternatively, the administration amount can be selected, for example, in the range of about 0.00001 to about 100000 mg/body per patient. When a cell secreting the peptide of the present application or a gene therapy vector having a DNA encoding the peptide inserted therein is administered, the cell or the vector can be administered such that the amount of the peptide is within the above range. However, the pharmaceutical composition of the present application is not limited to these administration amounts.

The pharmaceutical composition of the present application can be formulated according to conventional methods (e.g., Remington's Pharmaceutical Science, latest edition, Mark Publishing Company, Easton, U.S.A), and may contain a pharmaceutically acceptable carrier or an additive in addition to the peptide. Examples of the carrier and the additive include a surfactant, an excipient, a colorant, a fragrance, a preservative, a stabilizer, a buffer, a suspending agent, an isotonic agent, a binder, a disintegrant, a lubricant, a fluidity promoter, and a flavor modifier, but are not limited to these, and other conventional carriers and additives can be used as appropriate. Specific examples thereof include light anhydrous silicic acid, lactose, crystalline cellulose, mannitol, starch, carmellose calcium, carmellose sodium, hydroxypropyl cellulose, hydroxypropyl methyl cellulose, polyvinyl acetal diethylamino acetate, polyvinylpyrrolidone, gelatin, medium chain fatty acid triglyceride, polyoxyethylene cured castor oil 60, white sugar, carboxymethyl cellulose, corn starch, inorganic salts, purified water, physiological saline, and glycerin.

It should be noted that all the prior art literatures cited herein are incorporated herein as references.

The present invention is further illustrated by the following Examples, but the present invention is not limited thereto.

### Examples

### Example 1

### Evaluation of efficacy of HMGB1 fragment peptide on articular cartilage defect

### (1) Materials and Methods

### i) Preparation of agent

A peptide consisting of amino acid residues 1-44 (SEQ ID NO: 1) of human-derived HMGB1 protein was chemically synthesized by a solid-phase method. Hereinafter, the HMGB1 fragment peptide is referred to as HMGB1 peptide (1-44), and in the drawings corresponding to Examples, the peptide is simply indicated as "1-44".

### ii) Creation of cartilage defect model mouse

C57BL/6J mice (6-7 weeks old, male, wild type) were purchased from CLEA Japan, Inc., and then acclimated to animal facilities until they were 8 weeks old (weighing approximately 25 g at 8 weeks old). While the mice were under 1.5-2.0% (v/v) isoflurane inhalation anesthesia, shaving was performed on the lower extremity of the side to create a cartilage defect, and a longitudinal skin incision of approximately 1 cm was applied with scissors to the middle of the knee joint. An articular dissection of approximately 1 cm was performed under the microscope with a sharp blade (#11) in the parapatellar medial approach to dislocate the patella laterally. The femoral pulley was confirmed, and a 0.5 x 0.5 x 0.5 mm cartilage defect was created at the center of the pulley using a 0.5 mm diameter hand-turned drill (manufactured by MEISINGER USA, L.L.C.). Inside of the joint was washed with saline, then the articular capsule was continuously sutured with 8-0 vicryl to prevent patellar dislocation, and the skin was sutured with 5-0 nylon thread.

### iii) Administration of peptide

Cartilage defect model mice created as described above were divided into an HMGB1 peptide (1-44) administration group (n = 16) and a control group (n = 16). To the HMGB1 peptide (1-44) administration group, a solution of HMGB1 peptide (1-44) adjusted to a concentration of 1 µg/µL with saline as solvent was administered from the tail vein in an amount of 100 µL/mouse (4 mg/kg as the dose of peptide) immediately after the cartilage defect was created, and thereafter, the same dose was administered twice a week until 4 weeks after the cartilage defect was created (hereinafter also referred to simply as "post-operative"). To the control group, saline was administered from the tail vein in an amount of 100 µL to each mouse on the same schedule as in the HMGB1 peptide (1-44) administration group.

### iv) Evaluation of effects by administration of peptide

In both the HMGB1 peptide (1-44) administration group and the control group, four mice were sacrificed at each time point of 2, 4, 8 and 12 weeks post-operative, and the cartilage defect creation sites of knee joints were removed for microscopic observation and tissue staining. For microscopic observation, the status of cartilage tissue was evaluated by scoring the gross appearance according to the criteria shown in Table 1 below using the Wayne scoring system. In addition, sections of cartilage tissue were created for histological evaluation and subjected to safranin O staining, and the status of cartilage tissue was evaluated by calculating the Wakitani score from the tissue images according to the criteria shown in Table 2 below.

**[Table 1]**

| Wayne score | | |
|---|---|---|
| Gross appearance | | Grade |
| Coverage | | |
| | >75% filled | 4 |
| | 50-75% filled | 3 |
| | 25-50% filled | 2 |
| | <25% filled | 1 |
| | No filling | 0 |

| Neocartilage color | | |
|---|---|---|
| | Normal | 4 |
| | 25% yellow/brown | 3 |
| | 50% yellow/brown | 2 |
| | 75% yellow/brown | 1 |
| | 100% yellow/brown | 0 |

| Defect margins | | |
|---|---|---|
| | Invisible | 4 |
| | 25% circumference visible | 3 |
| | 50% circumference visible | 2 |
| | 75% circumference visible | 1 |
| | Entire circumference visible | 0 |

| Surface | | |
|---|---|---|
| Smooth/level with normal | | 4 |
| Smooth but raised | | 3 |
| Irregular 25-50% | | 2 |
| Irregular 50-75% | | 1 |
| Irregular 75% | | 0 |

**[Table 2]**

| Wakitani score | | |
|---|---|---|
| Category | | Score |
| Cell morphology | | |
| | Hyaline cartilage | 0 |
| Mostly hyaline cartilage | | 1 |
| Mostly fibrocartilage | | 2 |
| Mostly non-cartilage | | 1 |
| Only non-cartilage | | 0 |

| Matrix staining (metachromasia) | | |
|---|---|---|
| | Normal | 0 |
| | (compared with adjacent host cartilage) | |
| | Slightly reduced | 1 |
| | Markedly reduced | 2 |
| | No metachromatic stain | 3 |

| Surface regularity† | | |
|---|---|---|
| | Smooth(>3/4) | 0 |
| | Moderate (>1/2-3/4) | 1 |
| | Irregular(1/4-1/2) | 2 |
| | Severely irregular(<1/4) | 3 |

| Thickness of cartilage | | |
|---|---|---|
| | >2/3 | 0 |
| | 1/3-2/3 | 1 |
| | <1/3 | 2 |

| Integration of donor with adjacent host cartilage | | |
|---|---|---|
| | Both edges integrated | 0 |
| | One edge integrated | 1 |
| | Neither edge integrated | 2 |
| Total maximum | | 14 |

| | | |
|---|---|---|
| tTotal smooth region of regenerated cartilage compared with entire region of cartilage defect | | |

### (2) Results

### i) Microscopic observation (gross appearance)

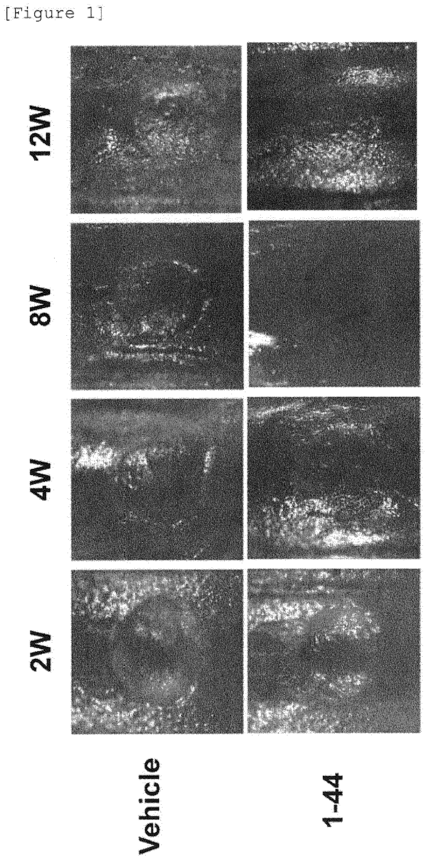

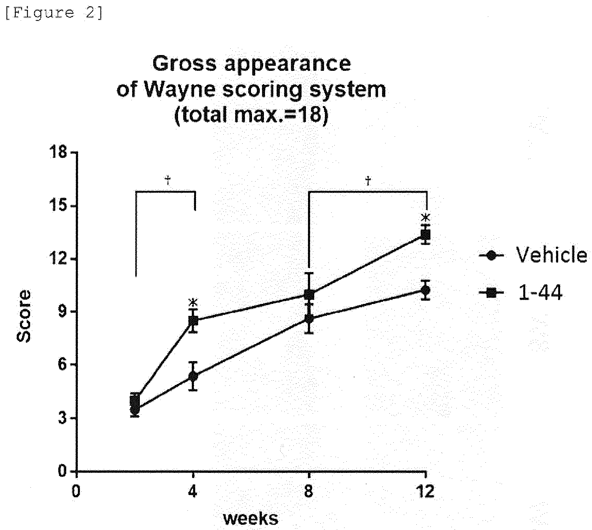

Figure 1 shows the results of microscopic observations at and around the cartilage defect sites of knee joints at 2, 4, 8 and 12 weeks post-operative. It was observed that the cartilage defect sites of the HMGB1 peptide (1-44) administration group were recovered to a state closer to normal than those of the control group. Also in the Wayne scores, the HMGB1 peptide (1-44) administration group was higher than the control group (Figure 2).

### ii) Histological evaluation

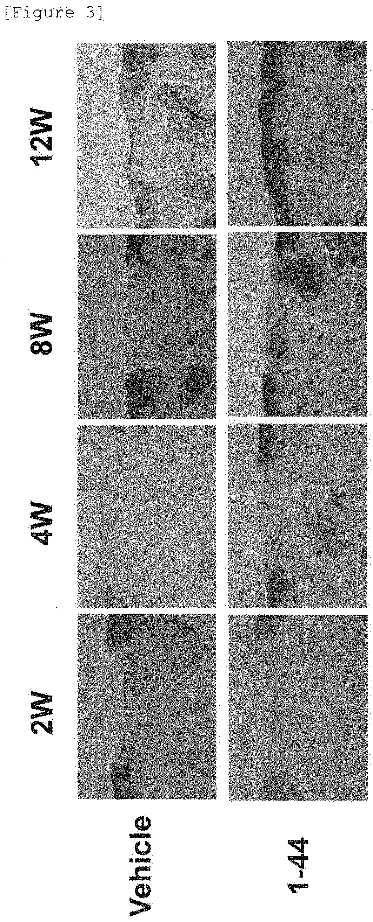

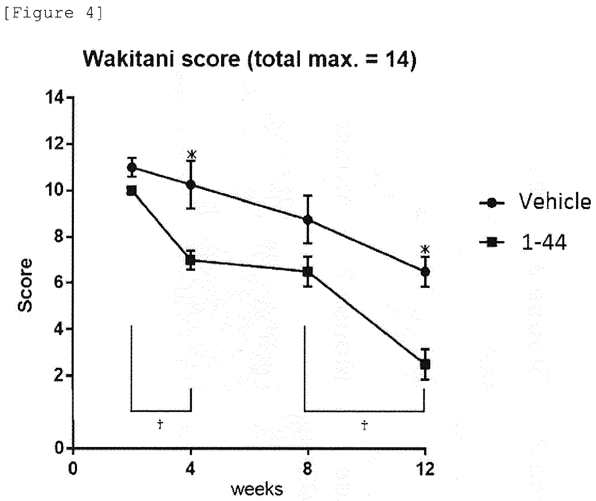

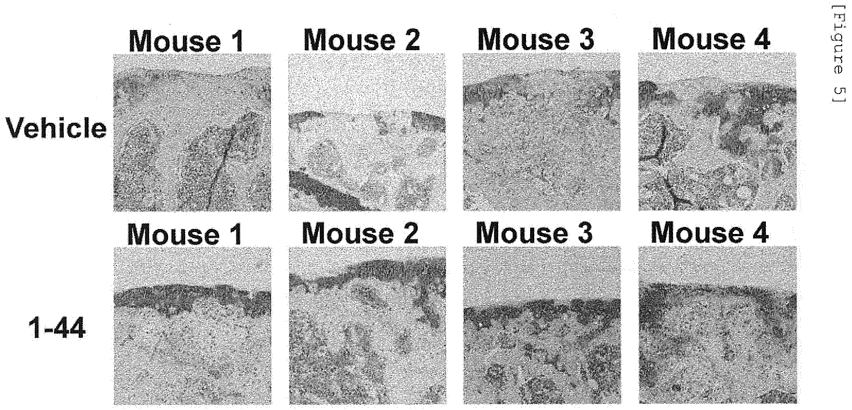

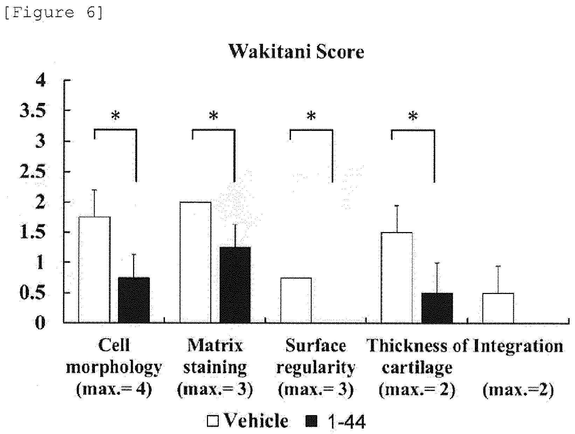

As a result of the safranin O staining of cartilage tissue sections, regions strongly stained with red (considered hyaline cartilage) were observed throughout the cartilage defect creation sites in the HMGB1 peptide (1-44) administration group, confirming that the defective cartilage tissue was fully regenerated (Figures 3 and 5). Evaluation by the Wakitani score also showed that the value of the HMGB1 peptide (1-44) administration group was significantly lower compared to that of the control group, showing the therapeutic effect of the HMGB1 peptide on cartilage defects of joints (Figures 4 and 6).

### Example 2

### Tissue staining of articular cartilage

### Methods

Cartilage defect model mice were created in the same manner as in Example 1 and were bred continuously. Tissues containing the cartilage defect creation sites of knee joints were removed from the mice at 12 weeks post-operative to create paraffin sections, and the sections were subjected to immunostaining with anti-type II collagen antibodies and safranin O staining. In addition, cartilage tissues of knee joints were removed from normal mice of the same strain to create paraffin sections, and the sections were subjected to immunostaining of type II collagen and safranin O staining in the same way.

### Results

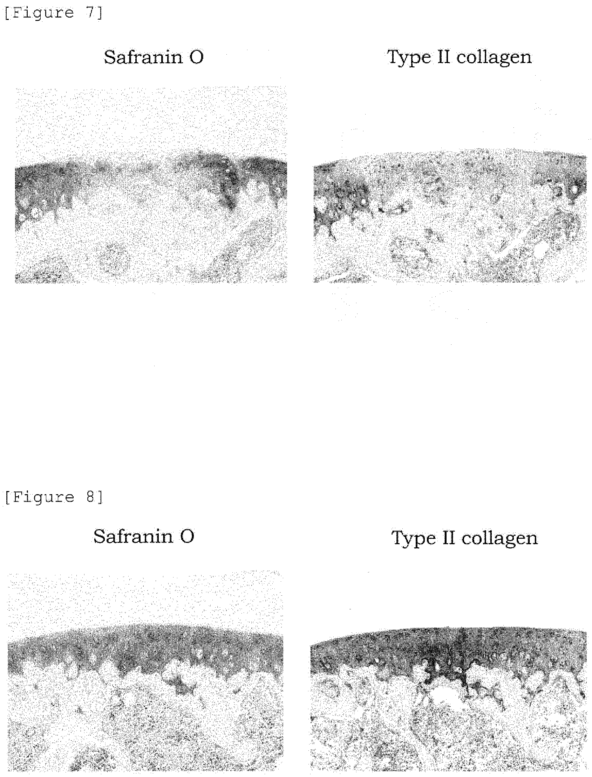

Tissues of cartilage defect creation sites of knee joints at 12 weeks post-operative in the cartilage defect model mice (i.e., tissues left to natural recovery after creation of cartilage defects) showed little positive response in either safranin O staining or immunostaining of type II collagen, thus confirming that they were not hyaline cartilage (Figure 7).

In contrast, cartilage tissues of knee joints of normal mice (known to be hyaline cartilage) showed a significantly stronger positive response in both safranin O staining and immunostaining of type II collagen compared to the cartilage defect creation sites at 12 weeks post-operative in the cartilage defect model mice described above (Figure 8). It was also confirmed that the positive regions in the safranin O staining and the positive regions in the type II collagen immunostaining corresponded well.

### Discussion

Cartilage tissues of knee joints (Figures 3 and 5) at 12 weeks post-operative in the HMGB1 peptide (1-44) administration group described in Example 1 show a strong positive response similar to cartilage tissues of knee joints of normal mice (Figure 8, left) in safranin O staining. Accordingly, the cartilage tissue regenerated by administration of the HMGB1 peptide (1-44) is believed to be a tissue equivalent to the normal articular cartilage tissue composed of hyaline cartilage.

### Industrial applicability

Since the pharmaceutical composition containing the peptide of the present application can regenerate a complete form of articular cartilage that cannot be regenerated naturally, it is expected that the pharmaceutical composition provides significant benefits for patients with cartilage disorders who don't have sufficient effects with existing therapeutic agents.

## Claims

1. A substance described in any of (a) to (c) below:
(a) a peptide consisting of an amino acid sequence set forth in SEQ ID NO: 1;
(b) a peptide consisting of an amino acid sequence set forth in SEQ ID NO: 1 with one to ten amino acids substituted, deleted, inserted, or added, wherein the peptide is functionally equivalent to the peptide according to (a) in that it is capable of regenerating cartilage tissue; and
(c) a peptide consisting of an amino acid sequence having a sequence identity of about 80% or more to the amino acid sequence set forth in SEQ ID NO: 1, wherein the peptide is functionally equivalent to the peptide according to (a) in that it is capable of regenerating cartilage tissue,
for use in preventing and/or treating a cartilage disorder, wherein the cartilage disorder is not psoriatic arthritis.

2. The substance for use according to claim 1, wherein the cartilage disorder is traumatic cartilage defect, osteoarthritis, or osteochondritis dissecans.

3. A substance described in any of (a) to (c) below:
(a) a peptide consisting of an amino acid sequence set forth in SEQ ID NO: 1;
(b) a peptide consisting of an amino acid sequence set forth in SEQ ID NO: 1 with one to ten amino acids substituted, deleted, inserted, or added, wherein the peptide is functionally equivalent to the peptide according to (a) in that it is capable of regenerating cartilage tissue; and
(c) a peptide consisting of an amino acid sequence having a sequence identity of about 80% or more to the amino acid sequence set forth in SEQ ID NO: 1, wherein the peptide is functionally equivalent to the peptide according to (a) in that it is capable of regenerating cartilage tissue,
for use in regenerating hyaline cartilage in a patient with a cartilage disorder.

4. The substance for use according to claim 3, wherein the cartilage disorder is traumatic cartilage defect, osteoarthritis, or osteochondritis dissecans.

5. A pharmaceutical composition for use in preventing and/or treating a cartilage disorder, comprising any one substance of (a) to (c):
(a) a peptide consisting of an amino acid sequence set forth in SEQ ID NO: 1;
(b) a peptide consisting of an amino acid sequence set forth in SEQ ID NO: 1 with one to ten amino acids substituted, deleted, inserted, or added, wherein the peptide is functionally equivalent to the peptide according to (a) in that it is capable of regenerating cartilage tissue; and
(c) a peptide consisting of an amino acid sequence having a sequence identity of about 80% or more to the amino acid sequence set forth in SEQ ID NO: 1, wherein the peptide is functionally equivalent to the peptide according to (a) in that it is capable of regenerating cartilage tissue,
wherein the cartilage disorder is not psoriatic arthritis.

6. The pharmaceutical composition for use according to claim 5, wherein the cartilage disorder is traumatic cartilage defect, osteoarthritis, or osteochondritis dissecans.

7. A pharmaceutical composition for use in regenerating hyaline cartilage in a patient with a cartilage disorder, comprising any one substance of (a) to (c):
(a) a peptide consisting of an amino acid sequence set forth in SEQ ID NO: 1;
(b) a peptide consisting of an amino acid sequence set forth in SEQ ID NO: 1 with one to ten amino acids substituted, deleted, inserted, or added, wherein the peptide is functionally equivalent to the peptide according to (a) in that it is capable of regenerating cartilage tissue; and
(c) a peptide consisting of an amino acid sequence having a sequence identity of about 80% or more to the amino acid sequence set forth in SEQ ID NO: 1, wherein the peptide is functionally equivalent to the peptide according to (a) in that it is capable of regenerating cartilage tissue.

8. The pharmaceutical composition for use according to claim 7, wherein the cartilage disorder is traumatic cartilage defect, osteoarthritis, or osteochondritis dissecans.

## Patentansprüche

1. Eine Substanz, die in einem der untenstehenden Punkte (a) bis (c) beschrieben ist:
(a) ein Peptid, bestehend aus einer Aminosäuresequenz, die in SEQ ID NO: 1 angegeben ist;
(b) ein Peptid, bestehend aus einer Aminosäuresequenz, die in SEQ ID NO: 1 angegeben ist, wobei ein bis zehn Aminosäuren substituiert, deletiert, insertiert oder hinzugefügt sind, wobei das Peptid insofern funktionell äquivalent zu dem Peptid gemäß (a) ist, als dass es in der Lage ist, Knorpelgewebe zu regenerieren; und
(c) ein Peptid, bestehend aus einer Aminosäuresequenz mit einer Sequenzidentität von etwa 80 % oder mehr zu der Aminosäuresequenz, die in SEQ ID NO: 1 angegeben ist, wobei das Peptid insofern funktionell äquivalent zu dem Peptid gemäß (a) ist, als dass es in der Lage ist, Knorpelgewebe zu regenerieren,
zur Verwendung bei der Vorbeugung und/oder Behandlung einer Knorpelerkrankung, wobei die Knorpelerkrankung keine Psoriasis-Arthritis ist.

2. Die Substanz zur Verwendung nach Anspruch 1, wobei die Knorpelerkrankung ein traumatischer Knorpeldefekt, Osteoarthritis oder Osteochondritis dissecans ist.

3. Eine Substanz, die in einem der untenstehenden Punkte (a) bis (c) beschrieben ist:
(a) ein Peptid, bestehend aus einer Aminosäuresequenz, die in SEQ ID NO: 1 angegeben ist;
(b) ein Peptid, bestehend aus einer Aminosäuresequenz, die in SEQ ID NO: 1 angegeben ist, wobei ein bis zehn Aminosäuren substituiert, deletiert, insertiert oder hinzugefügt sind, wobei das Peptid insofern funktionell äquivalent zu dem Peptid gemäß (a) ist, als dass es in der Lage ist, Knorpelgewebe zu regenerieren; und
(c) ein Peptid, bestehend aus einer Aminosäuresequenz mit einer Sequenzidentität von etwa 80 % oder mehr zu der Aminosäuresequenz, die in SEQ ID NO: 1 angegeben ist, wobei das Peptid insofern funktionell äquivalent zu dem Peptid gemäß (a) ist, als dass es in der Lage ist, Knorpelgewebe zu regenerieren,
zur Verwendung bei der Regeneration von hyalinem Knorpel bei einem Patienten mit einer Knorpelerkrankung.

4. Die Substanz zur Verwendung nach Anspruch 3, wobei die Knorpelerkrankung ein traumatischer Knorpeldefekt, Osteoarthritis oder Osteochondritis dissecans ist.

5. Eine pharmazeutische Zusammensetzung zur Verwendung bei der Vorbeugung und/oder Behandlung einer Knorpelerkrankung, umfassend eine der Substanzen (a) bis (c):
(a) ein Peptid, bestehend aus einer Aminosäuresequenz, die in SEQ ID NO: 1 angegeben ist;
(b) ein Peptid, bestehend aus einer Aminosäuresequenz, die in SEQ ID NO: 1 angegeben ist, wobei ein bis zehn Aminosäuren substituiert, deletiert, insertiert oder hinzugefügt sind, wobei das Peptid insofern funktionell äquivalent zu dem Peptid gemäß (a) ist, als dass es in der Lage ist, Knorpelgewebe zu regenerieren; und
(c) ein Peptid, bestehend aus einer Aminosäuresequenz mit einer Sequenzidentität von etwa 80 % oder mehr zu der Aminosäuresequenz, die in SEQ ID NO: 1 angegeben ist, wobei das Peptid insofern funktionell äquivalent zu dem Peptid gemäß (a) ist, als dass es in der Lage ist, Knorpelgewebe zu regenerieren,
wobei die Knorpelerkrankung keine Psoriasis-Arthritis ist.

6. Die pharmazeutische Zusammensetzung zur Verwendung nach Anspruch 5, wobei die Knorpelerkrankung ein traumatischer Knorpeldefekt, Osteoarthritis oder Osteochondritis dissecans ist.

7. Eine pharmazeutische Zusammensetzung zur Verwendung bei der Regeneration von hyalinem Knorpel bei einem Patienten mit einer Knorpelerkrankung, umfassend eine der Substanzen (a) bis (c):
(a) ein Peptid, bestehend aus einer Aminosäuresequenz, die in SEQ ID NO: 1 angegeben ist;
(b) ein Peptid, bestehend aus einer Aminosäuresequenz, die in SEQ ID NO: 1 angegeben ist, wobei ein bis zehn Aminosäuren substituiert, deletiert, insertiert oder hinzugefügt sind, wobei das Peptid insofern funktionell äquivalent zu dem Peptid gemäß (a) ist, als dass es in der Lage ist, Knorpelgewebe zu regenerieren; und
(c) ein Peptid, bestehend aus einer Aminosäuresequenz mit einer Sequenzidentität von etwa 80 % oder mehr zu der Aminosäuresequenz, die in SEQ ID NO: 1 angegeben ist, wobei das Peptid insofern funktionell äquivalent zu dem Peptid gemäß (a) ist, als dass es in der Lage ist, Knorpelgewebe zu regenerieren.

8. Die pharmazeutische Zusammensetzung zur Verwendung nach Anspruch 7, wobei die Knorpelerkrankung ein traumatischer Knorpeldefekt, Osteoarthritis oder Osteochondritis dissecans ist.

## Revendications

1. Substance décrite dans l'un des points (a) à (c) ci-dessous :
(a) un peptide constitué d'une séquence d'acides aminés présentée dans la SEQ ID N° 1 ;
(b) un peptide constitué d'une séquence d'acides aminés présentée dans la SEQ ID N° 1 avec un à dix acides aminés substitués, supprimés, insérés ou ajoutés, dans lequel le peptide est fonctionnellement équivalent au peptide conformément au point (a) en ce qu'il est capable de régénérer le tissu cartilagineux ; et
(c) un peptide constitué d'une séquence d'acides aminés présentant une identité de séquence d'environ 80 % ou plus avec la séquence d'acides aminés présentée dans la SEQ ID N° 1, dans lequel le peptide est fonctionnellement équivalent au peptide conformément au point (a) en ce qu'il est capable de régénérer le tissu cartilagineux,
destinée à être utilisée dans la prévention et/ou le traitement d'une pathologie liée au cartilage, dans lequel la pathologie liée au cartilage n'est pas l'arthrite psoriasique.

2. Substance destinée à être utilisée selon la revendication 1, dans laquelle la pathologie liée au cartilage est une lésion cartilagineuse traumatique, l'arthrose ou l'ostéochondrite disséquante.

3. Substance décrite dans l'un des points (a) à (c) ci-dessous :
(a) un peptide constitué d'une séquence d'acides aminés présentée dans la SEQ ID N° 1 ;
(b) un peptide constitué d'une séquence d'acides aminés présentée dans la SEQ ID N° 1 avec un à dix acides aminés substitués, supprimés, insérés ou ajoutés, dans lequel le peptide est fonctionnellement équivalent au peptide conformément au point (a) en ce qu'il est capable de régénérer le tissu cartilagineux ; et
(c) un peptide constitué d'une séquence d'acides aminés présentant une identité de séquence d'environ 80 % ou plus avec la séquence d'acides aminés présentée dans la SEQ ID N° 1, dans lequel le peptide est fonctionnellement équivalent au peptide conformément au point (a) en ce qu'il est capable de régénérer le tissu cartilagineux,
destinée à être utilisée dans la régénération du cartilage hyalin chez un patient souffrant d'une pathologie liée au cartilage.

4. Substance destinée à être utilisée selon la revendication 3, dans laquelle la pathologie liée au cartilage est une lésion cartilagineuse traumatique, l'arthrose ou l'ostéochondrite disséquante.

5. Composition pharmaceutique destinée à la prévention et/ou au traitement d'une pathologie liée au cartilage, comprenant l'une des substances des points (a) à (c) :
(a) un peptide constitué d'une séquence d'acides aminés présentée dans la SEQ ID N° 1;
(b) un peptide constitué d'une séquence d'acides aminés présentée dans la SEQ ID N° 1 avec un à dix acides aminés substitués, supprimés, insérés ou ajoutés, dans lequel le peptide est fonctionnellement équivalent au peptide conformément au point (a) en ce qu'il est capable de régénérer le tissu cartilagineux ; et
(c) un peptide constitué d'une séquence d'acides aminés présentant une identité de séquence d'environ 80 % ou plus avec la séquence d'acides aminés présentée dans la SEQ ID N° 1, dans lequel le peptide est fonctionnellement équivalent au peptide conformément au point (a) en ce qu'il est capable de régénérer le tissu cartilagineux,
dans laquelle la pathologie liée au cartilage n'est pas l'arthrite psoriasique.

6. Composition pharmaceutique destinée à être utilisée selon la revendication 5, dans laquelle la pathologie liée au cartilage est une lésion cartilagineuse traumatique, l'arthrose ou l'ostéochondrite disséquante.

7. Composition pharmaceutique destinée à être utilisée dans la régénération du cartilage hyalin chez un patient souffrant d'une pathologie liée au cartilage, comprenant l'une quelconque des substances des points (a) à (c) :
(a) un peptide constitué d'une séquence d'acides aminés présentée dans la SEQ ID N° 1;
(b) un peptide constitué d'une séquence d'acides aminés présentée dans la SEQ ID N° 1 avec un à dix acides aminés substitués, supprimés, insérés ou ajoutés, dans lequel le peptide est fonctionnellement équivalent au peptide conformément au point (a) en ce qu'il est capable de régénérer le tissu cartilagineux ; et
(c) un peptide constitué d'une séquence d'acides aminés présentant une identité de séquence d'environ 80 % ou plus avec la séquence d'acides aminés présentée dans la SEQ ID N° 1, dans lequel le peptide est fonctionnellement équivalent au peptide conformément au point (a) en ce qu'il est capable de régénérer le tissu cartilagineux.

8. Composition pharmaceutique destinée à être utilisée selon la revendication 7, dans laquelle la pathologie liée au cartilage est une lésion cartilagineuse traumatique, l'arthrose ou l'ostéochondrite disséquante.
